# EUROPEAN PATENT APPLICATION

(11) **EP 1 375 662 A1**
(43) Date of publication of application: **02.01.2004**
(21) Application number: 02704549.1
(22) Date of filing: 28.02.2002
(51) Int. Cl.: C12N 15/12, C12N 15/19, C07K 14/515

(54) **A NOVEL POLYPEPTIDE COMPOSE SUBSTANCE FOR PROMOTING BLOOD AND BLOOD VESSEL CELL GROWTH**

(30) Priority: 28.02.2001 CN 01109083
(71) Applicant: Institute of Hematology & Blood Hospital Chinese Academy of Medical Sciences, Tianjin 30002 (CN)
(72) Inventor: HAN, Zhongchao, Inst. Hematology & Blood Hospital, Tianjin 30002 (CN); Liu, Yongjun, Inst. Hematology & Blood Hospital, Tianjin 30002 (CN); Cai, Yinlin, Inst. Hematology & Blood Hospital, Tianjin 30002 (CN)
(74) Representative: Ganahl, Bernhard
(86) International application number: PCT/CN2002/000129
(87) International publication number: WO 2002/068642

(57) **Abstract**

The present invention relates to a novel stimulating factor which can promote the growth of hematopoietic stem progenitor cells and vascular endothelial cells, i.e. hemangiopoietin (HAPO), characterized in that design a variety of primers based on known sequences, obtain a series of gene segments through PCR amplification, clone into prokaryotic expression vector using double-enzyme restrict sites, in which two fragments of 879 bp and 558 bp were expressed in engineered bacterium, HAPO of 31.8KD and 20.1KD (292 and 186 amino acids) were obtained through enzyme digestion, specific antibodies were obtained after purification and immunizing rabbit with it, the antibodies is a useful tool for clinical diagnosis and treatment of haemopoietic and vascular diseases.

## Description

### FIELD OF THE INVENTION

The present invention relates to a novel stimulating factor, namely hemangiopoietin (abbreviated as HAPO), which can promote the growth of hematopoietic stem cells and vascular endothelial cells. The present invention relates, more particularly, to the cloning and expression of HAPO and its reconstitutant cDNA, the preparation and purification of recombinant HAPO protein, to the preparation of polyclonal and monoclonal antibodies against HAPO, and to the biological functions of HAPO in vitro and in vivo etc.

### BACKGROUND OF THE INVENTION

Human peripheral blood cells are generally short lived and are constitutively generated from hematopoietic stem cells present in bone marrow. When organisms are under stress conditions, such as infection and injury, the activated T-cells will secret a series of cytokines capable of stimulating hematopoiesis. Hypoxia can induce production of erythrocytes through the elevation of erythropoietin (EPO) expression. The proliferation and differentiation of hematopoietic cells depend upon the stimulation of cytokines and the surface molecules of the bone marrow stromal cells. Without such stimulation, hematopoietic cells not only stop proliferation but also undergo apoptosis. In the past 20 years, a number of cytokines that stimulate proliferation and differentiation of hematopoietic cells have been identified and the intensive investigations about their working mechanism have been made.

Generally, peripheral blood cells can be classified into three major groups: erythrocytes, leucocytes, and platelets. After the pluripotent stem cells in the bone marrows differentiated into lymphoid stem cells or myeloid stem cells (CFU-GEMM), they would be induced further to differentiate into lymphocytes, erythrocytes, granulocytes (neutrophil, eosinophil, basophil), macrophage and megakaryocytes (platelet). Most cytokines relating to hematopoiesis are produced by stromal cells, T-cells, monocytes, macrophages and the likes. Normal hematopoiesis is regulated by cytokines secreted from stromal cells and the inducing hematopoiesis is regulated by activated T-cells. Interleukin-3 (IL-3), as also known as multicolony-stimulating factor, stimulates the proliferation of pluripotent hematopoietic stem cells as well as various lineage-committed progenitors. Granulocyte-macrophage (GM)-CSF also stimulates early progenitors in addition to granulocytes and macrophage. In contrast, EPO, G-CSF, M-CSF, and IL-5 predominantly stimulate erythroid, granulocytes, monocytes/macrophages, and eosinophils, respectively. Originally, it was believed that TPO promotes proliferation and differentiation of megakaryocytes as a lineage-specific cytokine. However, recent research indicates that it has broader targets including multipotential progenitors. IL-6, IL- 11, IL-12, leukemia inhibitory factor (LIF) and stem cells factor (SCF) are very important for the proliferation and self-renewal of hematopoietic stem cells. The identification of other cytokines is a major subject of current hematology.

Modem biomedical researches have suggested that hematopoietic cells and vascular cells share a common precursor, i.e. hemangioblast, during the embryonic development. The cytokines promoting the proliferation of hemangioblast should be those capable of stimulating both hematopoietic and vascular endothelial cells. We have isolated and identified a type of cytokine―HAPO from the urine of the patients with aplasitic anemia, which has the stimulating effect on hematopoietic cells. The expression of HAPO in the mRNA level is detected by RT-PCR. We found that HAPO is expressed mainly in human embryonic liver and spleen. It is presumed that HAPO could be an early stage protein of embryonic development. Experimental results suggested that it could promote the proliferation of hematopoietic stem/progenitor cells and vascular endothelial cells.

### DISCLOSURE OF THE INVENTION

An object of the present invention is directed to a novel cytokine derived and identified from the serum and urine of patients with aplastic anemia, which can promotes the proliferation of cells. The bone marrow experiments of mice in vivo and vitro and the analysis of human vascular endothelial cells and bone marrow cultured in vitro indicated that the cytokine has the dual functions for promoting the propagation of hematopoietic stem cells and vascular endothelial cells. It is designated as hemangiopoietin (referred to as HAPO hereafter) based on its biological functions.

Another object of the present invention is directed to cloning and reconstituting a set of HAPO polypeptide compositions by PCR amplification and mutation methods based on known sequences, to establishing a set of prokaryocetic expression systems for HAPO, to selecting and producing an active recombinant human HAPO.

Another object of the present invention is directed to establishing a set of purification methods for HAPO.

A further object of the present invention is directed to preparation of the polyclonal and monoclonal antibodies against HAPO.

In the examples of the present invention, we designed various primer pairs based on known sequences for PCR amplification to obtain a series of gene fragments, cloned them into the reconstituted prokaryocetic expression vector pET32c (+) utilizing BamHI and HindIII double restriction sites, and constructed a series of expression vectors pET32c-HAPOs. Among them, 558bp and 879bp fragments achieved effective and soluble expression as fusion form in the engineered strains BL21 and obtained a pure HAPO protein free of foreign amino acids by enterokinase digestion. HAPO with high activities was obtained by HAPO recombinants and mutants of the present invention, including the expressing fragments having selectively active expression regions and having several amino acid sites mutated directionally/randomly by analysis of amino acid sequence domains of HAPO.

The HAPO purification methods of the present invention include ion exchange chromatography (DEAE ion exchange column, SP ion exchange column), affinity chromatography and conventional sterilizing filtration, concentration, lyophilization and the like.

In the examples of the present invention, rabbits were immunized four times with purified HAPO by SDS-PAGE and then the specific rabbit anti-rh-HAPO polyclonal antibodies were successfully obtained. The result of double diffusion was positive, the dilution was 1: 8. Western blot analysis showed that said antibodies specifically recognize recombinant human HAPO proteins.

### THE IMPORTANT APPLICATIONS OF THE INVENTION

(1)According to present invention, the cloning HAPO gene fragments, the establishing series of HAPO engineered strains, the producing recombinant HAPO and its variants by expression will provide ideas and tools for investigating the structure and function of HAPO.
(2) HAPO and its recombinants can be served as blood and vascular growth factors, applied in the study of the growth regulation of hematopoiesis and angiogenesis, and can be served as the important agents for the amplification of propagating hematopoietic stem cells, progenitor cells in vitro and the like
(3) HAPO proteins or active polypeptides or muteins thereof combined with some haemopoietic factors (IL-3, SCF , EPO, TPO and SCF etc.) can be used to treat certain haemopoietic diseases.
(4) The promoting effect of HAPO on vascular endothelial cells makes it become a promising gene therapy drug for treating cardiovascular diseases.
(5) HAPO and the antibodies thereof can be used as an important tool for searching HAPO receptor, which can be carried out by currently conventional methods for receptor identification.

### Description of the drawings

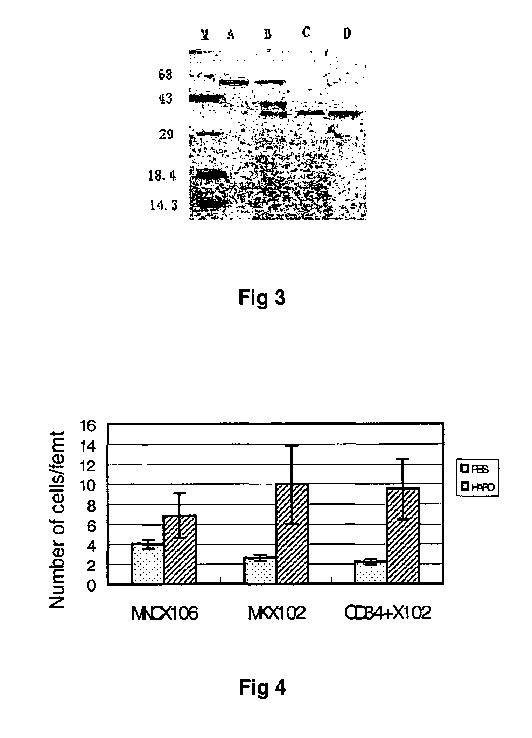

Figure 1. The construction of prokaryocytic expression vectors
Figure 2. The expression of HAPO
   2a: The expression of 558bpHAPO , in which, M: protein standards, 1: pET―HAPO558, non-induced, 2: pET―HAPO558, induced, 3, 4: purified HAPO
   2b: The expression of 876bpHAPO, in which, M: protein standards, 1: empty vector, non-induced, 2: empty vector, induced, 3: pET―HAPO876, non-induced, 4: pET―HAPO876, induced
Figure 3. The restriction map of thrombin, in which, M: standard molecular weights, A: no thrombin, B: digested with thrombin for 8 hours at 25°C , C: digested for 12 hours with thrombin at 25 °C, D: purified HAPO
Figure 4. The numbers of monocytes (MNC), megakaryocytes (MK) and CD34-positive stem/ progenitor cells in thighbone

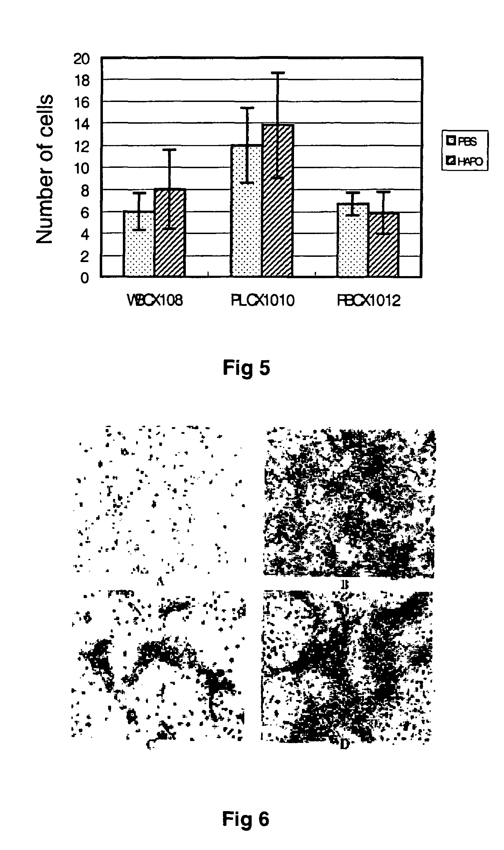

Figure 5. The numbers of platelets (PLT), white blood cells (WBC) and red blood cells (RBC) in the peripheral blood
Figure 6. The promoting effect of HAPO on the propagation of mouse bone marrow cell, in which, A: control , B: added HAPO, C: added porcine refractory serum, D: added porcine refractory serum plus HAPO

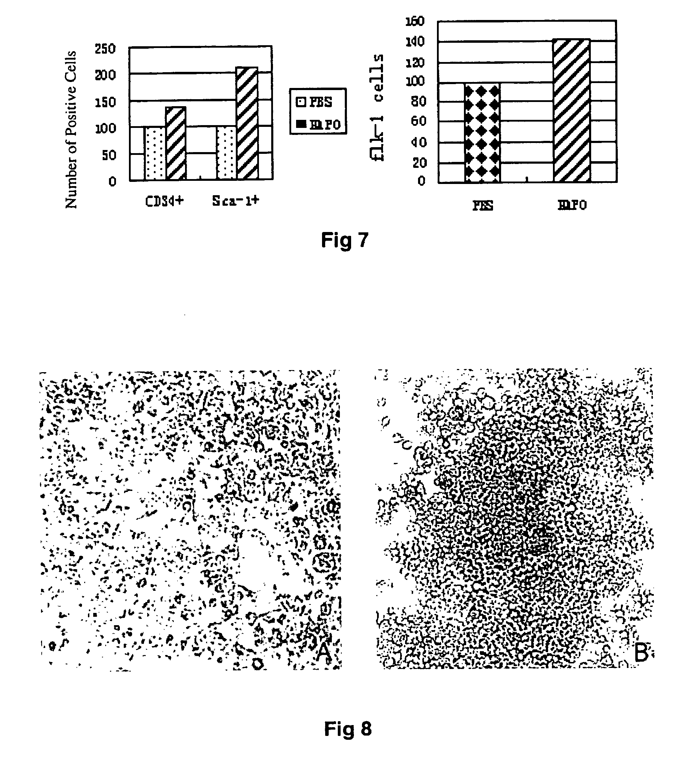

Figure 7. The effects of HAPO on CD34⁺, Sca― 1 and flk― 1 in mouse bone marrow cells, in which, Figure 7a represents the effect of HAPO on long term cultured mouse bone marrow cells, Figure 7b represents the effect of HAPO on long term cultured mouse endothelial cells
Figure 8. The effect of HAPO on long term cultured mouse bone marrow initiation cells (LTC―IC), in which, A: control, B: Added HAPO

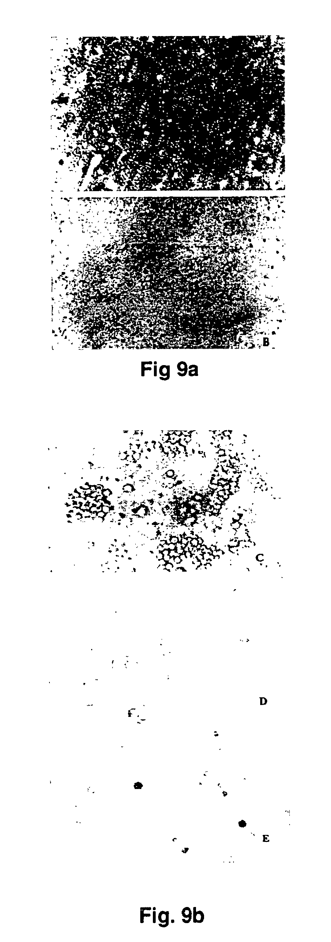

Figure 9a. The effect of HAPO on liquid cultures of human bone marrow cells, in which, A: Added HAPO and incubated 3 weeks to form a big colony, B : control
Figure 9b. The effect of HAPO on colony formation of human bone marrow cells, in which, C , D: colonies formed after 14 days when HAPO added, E: control

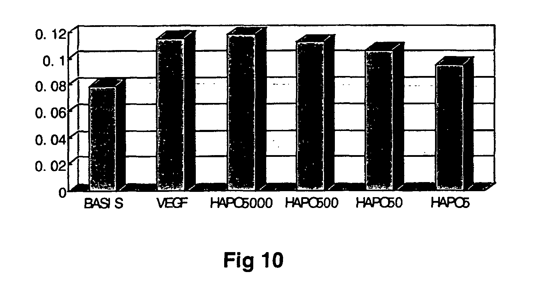

Figure 10. The effect of HAPO on the propagation of HUVECs

### THE BEST MODES FOR CARRYING OUT THE INVENTION

The present invention is further described by following examples hereinafter however, the present invention is not restricted to these examples.

### Example 1. The reconstitution of pET-32c (+) vector

In order to adapt the expression of HAPO gene, we have reconstituted pET-32c (+) vector. After removed thioredoxin (Trx.Tag) between *Mscl* and *BamHI* restriction sites from the vector, the enterokinase sequence was reconstituted into thrombin sequence, in order that the fused protein expressed can be cleaved by thrombin and can obtain original protein. That is the region between two restriction sites of *MscI* and *BamHI was* replaced with following sequence: 
——TGGCCAT ATG CAC CAT CAT CAT CAT CAT TCT GGT CTG GTT CCG
CGT GGA TCC ——
 The reconstituted vector can be used for further manipulations.

### Example2.The preparation of HAPO and its reconstitutant cDNA

Two pairs of PCR primers were synthesized based on the cDNA sequence of HAPO by computer assisted analysis and design, and were used to amplify different HAPO cDNA fragments containing restriction endonuclease recognizable site respectively, the amplified fragments were 558, 876bp respectively. The upstream primer used for amplifying the 558bp HAPO fragment was 5'-GCG GTA GGA TCC GAT GCC ACC TGC AAC TGT-3'; the downstream primer was 5'-AGC GTA AAG CTT CTA ATC AGA ATT AGG TGG AAG-3' . The upstream primer used for amplifying the 879bp HAPO fragment was 5'-GCG GTA GGA TCC GAT GCC ACC TGC AAC TGT-3': the downstream primer was 5'-CAG GTA AAG CTT CTA GGT GGG TGC AGA CTT GAT-3'. *BamHI* and *HindIII* restriction sites were introduced into the 5'-end and 3'-end of the two pairs of primers respectively. Amplification was performed with high fidelity Pfu DNA polymerase using the HAPO cDNA stored in our laboratory as a template. PCR amplification was performed as follows: 94°C 5min; 94°C 45S, 65°C 1.5min, 72°C 3min: and finally elongated 10minutes at 72°C, cycling 30 cycles.

### Example3. The construction of expression vectors

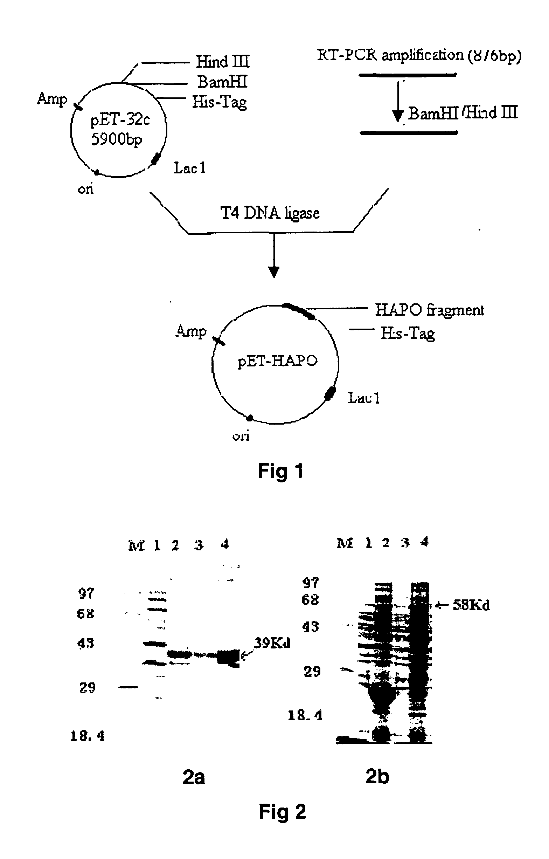

Molecular cloning was carried out following the methods described by Sambrook et al. (Sambrook, T. et al. Molecular Cloning: A Laboratory Manual, 1989, Cold Spring Harbor Laboratory, New York. PCR products and reconstituted pET32c vector were restricted double with *BamHI*/*HindIII* respectively, the corresponding bands were recovered with DE-81 filter, the ligation of inserted fragment and plasmid vector and transformation were carried out by conventional methods. Positive clones were identified through *BamHI* /*HindIII* double restriction, meanwhile send them to Shanghai Bioengineering Company for sequencing , the correct clones were used for expressing. The 876bp HAPO was presumed as a protein of 31.8Kd based upon amino acid sequence, plus about 17.4KD of the fusion protein at N-end, it was presumed that the expressed protein could be about 58.2KD. The 555bp HAPO was presumed as a protein of 20.1Kd based upon amino acid sequence , plus about 17.4KD of the fusion protein at N-end , it was presumed that the expressed protein could be about 37.5KD (see Fig.1).

### Example 4. The expression and SDS-PAGE characterization of recombinant proteins

A positive single colony was inoculated into 3ml LB medium containing ampicillin, after cultivating shakily at 37°C overnight , 30µl of said cultures was inoculated into 3ml LB medium containing ampicillin , after cultivating shakily 3hrs at 37°C , added IPTG make the final concentration be 1mmol/L, induced for 6hrs, then harvested 1 ml bacteria liquid, centrifugalized, and added 50 µl 2XSDS loading buffer, boiled 10 minutes at 100°C. took 5 µl for applying for detecting the proteins expressed through 12% SDS-PAGE, and stained with Coomassie brilliant blue. The expression level of recombinant protein was detected through scanning with the UltroScan XL laser optical density scanner supplied by LKB Company. The experimental results indicated that the expression of recombinant plasmid pET-HAPO in E. coli was achieved, the 558bp fragment is able to express a fusion protein of 39Kd and its desired expressed protein comprises 30-35% of the total protein of the bacteria, and the 876bp fragment is able to express a 58Kd fusion protein and its desired expressed protein comprises 10% of the total protein of the bacteria, as shown in Fig.2.

The soluble or non-soluble detection of recombinant protein : 100ml of induced bacteria liquid was sampled , centrifugalized at 7000 rpm for 10 minutes, removed the supernatant, suspended in 1/10 volume (10ml) of solution containing 50mmol/L Tris-HCl pH8.0, 2mmol/EDTA, added protease inhibitor PMSF make the final concentration be 1 µ mol/L, placed on ice, the bacteria were sonicated to the no -viscous form. Centrifugalized 15 minutes at 12000 rpm at 4°C. The supernatant was soluble protein and the pellet contained non-soluble inclusions. 300 µ l aliquots of the bacteria liquid were sampled respectively and characterized through 12% SDS-PAGE . The results showed that the soluble expression is more than 95%.

### Example 5. The preparation and purification of recombinant human HAPO

### The preparation of HAPO fusion protein

After inductive expression, the bacteria were sonicated, centrifuged 15minutes at 12000rpm at 4°C, fractioned roughly with DEAE anion exchange column at first, removed 1/3 of contaminant proteins, lipids and saccharides etc, to prevent the nickel column from contamination. The recombinant proteins were Purified with Chelating Sepharose Fast Flow supplied by Pharmacia company, equilibrated the nickel column, loaded nickel, and washed following the instructions of the manufacture. The roughly fractionated sample was applied into the nickel ion affinity chromatography column, allow the desired protein of fusion form binding to the column, after washing, the column was eluted with 6 times of bed volume of Tris-HCl buffer containing 120mM imidazole. The elutant was dialyzed against 50mM Tris-HCl buffer at pH7.8 for 48 hours. The concentration of the protein was determined and prepared for enzyme digestion.

### THE LYSATES OF HAPO FUSION PROTEIN

The fusion protein was lysed with thrombin, the ratio of the enzyme to fusion protein is 1: 100, and digested 12 hours at 25°C, purify the lysates through the nickel column, the HAPO protein free of foreign amino acids was obtained , and purified through SP ion column further. After purifying , 1mg HAPO protein (876bp fragment expressing products) can be obtained from one liter of broth. The pure HAPO (100µg/vial) was produced through sterilizing filtration, protein quantification, dispensation, and lyophilization. See Fig.3.

### Example 6. The preparation of recombinant human HAPO polyclonal antibodies

4-6 white rabbits of fine breed with big ears were selected, raised for their adaptability, observed their health for one week. The rabbits were basically immunized with conventional BCG(Bacille de Calmette Guerin). An amount of antigen SDS-PAGE was stained with Coomassie brilliant blue (or staining with 250Mm KCl, dissected the desired bands, pestled the gel in mortar with liquid nitrogen, mixed the gel and adjuvant (1: 1) and homogenized continuously it into paste which comprises 1mg antigen/g gel. The rabbits were immunized with 1―2mg antigen/rabbit at one week after the basic immunization. Furthermore:
1. having a booster immunization with 1-2mg antigen/ rabbit after two (or three) weeks.
2. having a booster immunization again with 1―2mg antigen/ rabbit after one week, monitored whether the antibodies is produced or not through dimensional diffusion method.
3. having the third booster immunization with 1―2mg antigen/ rabbit after one week.

The blood from the ear edge of the rabbits was collected after two weeks, detected anti-HAPO antibodies by agar dimensional diffusion method, and the antibody titer was measured by ELISA. After the desired antibodies in serum became positive, a large amount of rabbit blood was withdrawn. The collected blood was deposited 1-2 hours at 37°C and incubated at 4°C overnight. The serum was collected by centrifugation under sterile conditions, dispensed (0.2ml), and stored at -70°C.

### Example 7. The effects of HAPO on the reconstitution of mouse bone marrow after radiation

Balb/c mouse was radiated with cesium source under 4.5Gy and divided into 2 groups randomly. The mouse of experimental group were injected with a dosage 15µg HAPO / mouse each day for 10 days: and the mouse of control group were injected with PBS. The peripheral blood was taken for measuring the numbers of platelets, leucocytes and erythrocytes at day 12 after radiation. Then the mouse was sacrificed by cervical vertebra dislocation, taken the thighbone and got bone marrow cells which were carried out the following measurements: measuring the numbers of monocytes (MNC) in the bone marrow, measuring the numbers of megakaryocytes (MK) through spread staining, measuring the numbers of MK and megakaryocytic progenitor cells (CFU-MK) through liquid and semiliquid methods, detecting the content of CD34 positive cells in bone marrow through FACS .

Results: MNC in the bone marrow of experimental mouse group increased by 1.7 folds compared with that of the control group, and MK increased by 3.8 folds compared with that of the control group, CD34 positive cells increased by 4.3 folds compared with that of the control group (Fig.4) : platelets and leucocytes in peripheral blood increased slightly compared with the control group (Fig.5). The results suggested that HAPO have the abilities of stimulating the growth of hematopoietic cells and promoting the reconstitution of mouse bone marrow.

### Example 8. The effects of HAPO on the growth of mouse bone marrow cells in liquid culture

The bone marrow cells of the femoral and tibial of BALB/C mouse was formulated into 2 × 10⁶cells/ml suspension and these cells were inoculated onto 24 wells plate in 0.5ml each well, within the medium is IMDM containing 10%FCS, 10%.FCS+5%Fig serum. HAPO factors were inoculated (500 ng/ml) and cultured in liquid at 37°C, 5%CO₂/95%. A half of the medium was changed into fresh once every 3.5 days. A portion of the cultured cells was stained with Swiss stains at week 4 and take pictures, the results were shown as in Fig. 6.

Compared with the control group, the number of cells in the experimental group with HAPO factors increased significantly, and HAPO has a synergism with porcine refractory serum. Another portion of the cultured cells was labeled with antibodies and determined the numbers of CD34⁺, Sca-1, flk-1 cells with flow cytometer, the results is shown as in Fig. 7. Compared with the control group, cell numbers of CD34⁺, Sca-1 and Flk-1 among these cells with HAPO increased significantly. The results suggested that HAPO not only has the ability of promoting the propagation of hemapoietic stem cells/progenitor cells, but also has the ability of promoting the propagation of bone marrow endothelial cells (Flk-1).

### Example 9. The effects of HAPO on the growth of initiation cells of mouse bone marrow cultivated for long term (LTC-IC)

Firstly, the stromal cell layer was established, in particular: the mouse bone marrow cells were inoculated into 24 wells plate( 1x10⁶cell/well), the medium was changed into fresh and the hematopoietic cells were removed after 3.5 days. All of the medium was aspirated out after the stromal cells grown to confluency. The stromal cells were radiated with cesium source at 1200cGy after washing 2 times with fresh medium and adding 1ml medium, then the medium was removed. The mouse bone marrow monocytes screened were inoculated(50000cell/well)onto the stromal cells layer radiated, incubated with 0.5ml medium containing 10% fetal calf serum , 10%equine serum , 0.1µM hydrocortisone succinate, 200mM glutamine in a CO₂ incubator, with 5%CO₂/95% air and 100% humidity, at 33°C. PBS control group and experimental group with HAPO factors were set respectively. A half of the medium was changed into fresh once every 3.5 days, incubated 5 weeks, removed the liquid medium, added the methyl cellulose medium containing EPO 3U/ml, GM-CSF 20ng/ml, G-CSF 20ng/ml, IL-3 20ng/ml, IL-6 20ng/ml, and SCF SOng/ml. After incubating continuously 14 days in a CO₂ incubator, with 5%CO₂/95% air and 100% humidity , at 33°C, the CFU colonies were counted under inverted microscope. The results are shown as in Fig. 8.

Results: After incubating continuously 14 days, 4-7 colonies were observed in each experimental group with 100ng/ml HAPO factors under the microscope respectively, while no colony was formed in the PBS control group. In Fig.8 A represents PBS control group, B represents one of colonies of the experimental group with HAPO factor.

### Example 10. The propagation promoting effect of HAPO on human bone marrow cells

The bone marrow cells were taken from normal people, the monocytes were separated with lymphocyte separating buffer and the cells were suspended in IMDM into 5 × 10⁴cells/ml suspension. After incubating for three weeks, the 1000 suspended cells of IL-3, SCF, GM-CSF and EPO were inoculated into 1 ml methylcellulose semi-solid medium respectively and the experimental groups were added HAPO additionally. They were incubated in a CO₂ incubator, with 5%CO₂/95% air and 100% humidity, at 37°C. CFU colonies were counted on 14^{th} day and results is shown as in Figure 9.

### Example 11. The effects of HAPO on human umbilical vein endothelial cells

The cultivation of human umbilical vein endothelial cells (HUVEC) was performed through modified Jaffe method^{[20]}. The fresh fetal umbilical cord (15∼ 25cm long) was taken, stored in sterile Hanks buffer at 4°C and incubated under digestive condition for 24hrs. The tubes were inserted into both ends of umbilical cord, the remaining blood of the umbilical vein was flushed out with PBS before 0.3% trypsin was injected into it. The both ends of the tubes were clamped with hemostat, then the umbilical cord was incubated 15∼17minutes in the water bath at 37°C. The digestive liquid was flushed with PBS into a centrifuge tube containing scrum, and centrifuged 10 minutes at 1000rpm to obtain HUVEC which was then added M199 medium (containing 20%FCS, 80IU/ml penicillin, 80IU/ml streptomycin, and 0.3mg/ml L-glutamine). It was seeded into flasks and incubated 24hrs in 5% CO₂ at 37°C, then the medium was changed into fresh. It was characterized by morphological observation under inverted microscope and VIII factor related antigen using indirect immunofluorescence assay at 2∼3 days after the cultured cells were fused into monolayer. The monolayer HUVECVs were digested with 0.25% trypsin and the suspension of single cell was prepared with M199 complete medium containing 20% FCS, which was inoculated onto 96 wells plate at 1x10⁴/well as subculture. The effects of HAPO on the propagation of HUVECs were assessed through MTT method. The 2^{nd} generation of HUVECs was incubated for 4hrs in 48hrs after subculture for 24 hrs, in the medium containing VEGF 100ng/ml, HAPO5000ng/ml, HAPO 500ng/ml, HAPO 50ng/ml, HAPO 5ng/ml, respectively, Then 20µl 5mg/ml MTT was added to each well at 37 °C and the supernatant in the wells was aspirated out and discarded. 150µl DMSO were added to each well, shaken 10 minutes to solubilize the crystals thoroughly, and determined the absorbance on an enzyme-linked immunodetector at 490nm calibrated to zero by referencing to the blank wells. The results is shown as in Fig. 10.

Conclusion: The results from the experiments in vivo and in vitro shown that HAPO has the ability to stimulate the growth of megakaryocytes, hematopoietic stem/progenitor cells and vascular endothelial cells.

Sequence listing

HAPO gene sequence (558bp) :
GATGCCACCTGCAACTGTGATTATAACTGTCAACACTACATGGAGTGCTGCCCTGATTTCAAGAGAGTCTGCACTGC
GGAGCTTTCCTGTAAAGGCCGCTGCTTTGAGTCCTTCGAGAGAGGGAGGGAGTGTGACTGCGACGCCCAATGTAAGA
AGTATGACAAGTGCTGTCCCGATTATGAGAGTTTCTGTGCAGAAGTGCATAATCCCACATCACCACCATCTTCAAAG
AAAGCACCTCCACCTTCAGGAGCATCTCAAACCATCAAATCAACAACCAAACGTTCACCCAAACCACCAAACAAGAA
GAAGACTAAGAAAGTTATAGAATCAGAGGAAATAACAGAAAAAGATAACAAGAAGAACAGAACTAAAAAGAAACCTA
CCCCCAAACCACCAGTTGTAGATGAAGCTGGAAGTGGATTGGACAATGGTGACTTCAAGGTCACAACTCCTGACACG
TCTACCACCCAACACAATAAAGTCAGCACATCTCCCAAGATCACAACAGCAAAACCAATAAATCCCAGACCCAGTCT
TCCACCTAATTCTGAT

Amino acid sequence: (186AA、20.1KD)
DATCNCDYNCQHYMECCPDFKRVCTAELSCKGRCFESFERGRECDCDAQCKKYDKCCPDYESFCAEVHNPTSPPSSK
KAPPPSGASQTIKSTTKRSPKPPNKKKTKKVIESEEITEKDNKKNRTKKKPTPKPPVVDEAGSGLDNGDFKVTTPDT
STTQHNKVSTSPKITTAKPINPRPSLPPNSD

HAPO gene sequence (879bp) :
GATGCCACCTGCAACTGTGATTATAACTGTCAACACTACATGGAGTGCTGCCCTGATTTCAAGAGAGTCTGCACTGC
GGAGCTTTCCTGTAAAGGCCGCTGCTTTGAGTCCTTCGAGAGAGGGAGGGAGTGTGACTGCGACGCCCAATGTAAGA
AGTATGACAAGTGCTGTCCCGATTATGAGAGTTTCTGTGCAGAAGTGCATAATCCCACATCACCACCATCTTCAAAG
AAAGCACCTCCACCTTCAGGAGCATCTCAAACCATCAAATCAACAACCAAACGTTCACCCAAACCACCAAACAAGAA
GAAGACTAAGAAAGTTATAGAATCAGAGGAAATAACAGAAAAAGATAACAAGAAGAACAGAACTAAAAAGAAACCTA
CCCCCAAACCACCAGTTGTAGATGAAGCTGGAAGTGGATTGGACAATGGTGACTTCAAGGTCACAACTCCTGACACG
TCTACCACCCAACACAATAAAGTCAGCACATCTCCCAAGATCACAACAGCAAAACCAATAAATCCCAGACCCAGTCT
TCCACCTAATTCTGATACATCTAAAGAGACGTCTTTGACAGTGAATAAAGAGACAACAGTTGAAACTAAAGAAACTA
CTACAACAAATAAACAGACTTCAACTGATGGAAAAGAGAAGACTACTTCCGCTAAAGAGACACAAAGTATAGAGAAA
ACATCTGCTAAAGATTTAGCACCCACATCTAAAGTGCTGGCTAAACCTACACCCAAAGCTGAAACTACAACCAAAGG
CCCTGCTCTCACCACTCCCAAGGAGCCCACGCCCACCACTCCCAAGGAGCCTGCATCTACCACACCCAAAGAGCCCA
CACCTACCACCATCAAGTCTGCACCCACC

Amino acid sequence: (293AA、31.8KD)
DATCNCDYNCQHYMECCPDFKRVCTAELSCKGRCFESFERGRECDCDAQCKKYDKCCPDYESFCAEVHNPTSPPSSK
KAPPPSGASQTIKSTTKRSPKPPNKKKTKKVIESEEITEKDNKKNRTKKKPTPKPPVVDEAGSGLDNGDFKVTTPDT
STTQHNKVSTSPKITTAKPINPRPSLPPNSDTSKETSLTVNKETTVETKETTTTNKQTSTDGKEKTTSAKETQSIEK
TSAKDLAPTSKVLAKPTPKAETTTKGPALTTPKEPTPTTPKEPASTTPKEPTPTTIKSAPT

## Claims

1. A hemangiopoietin (HAPO), wherein it is a protein or the active fragment thereof with the any one of the amino acid sequences shown in the sequence list.

2. A method for purifying HAPO, wherein the method includes nickel ion chelated chromatography and/or heparin column affinity chromatography.

3. cDNA molecules encoding HAPO, wherein said molecules comprise:
(A) Whole nucleotide sequence of any one of the nucleotide sequences shown in the sequence list; or
(B) Partial nucleotide sequences of any one of the nucleotide sequences shown in the sequence list; or
(C) Nucleotide sequences which can hybridize with the nucleotide sequences (A) or (B).

4. A method for cloning cDNA of HAPO, wherein said method includes the steps of:
1)Deducing the nucleotides corresponding to HAPO amino acids from said HAPO amino acid sequences using known genetic codes;
2) Designing specific primers for PCR amplification;
3) Selecting cDNA of HAPO from human cDNA library using the specific cDNA amplifying products as probes.

5. The method according to claim 4, wherein said human cDNA library is human embryonic liver cDNA library.

6. A plasmid for expressing HAPO, wherein it comprises said DNA molecules according to claim 3.

7. The plasmid according to claim 6, wherein said plasmid is selected from reconstituted pET32.

8. A pharmaceutical composition, wherein the composition. comprises HAPO and/or the mutant thereof.

9. The pharmaceutical composition according to claim 8, wherein further comprises cytokines and hematopoietic growth factors.

10. The pharmaceutical composition according to claim 9, wherein said cytokines and hematopoietic growth factors are selected from stem cell factors, interleukin 3, 6 and 11, erythropoietin, granulocyte and granulocyte colony form stimulating factor and platelet factor 4.

11. The pharmaceutical composition according to any one of claim 8-10, wherein further comprises polysaccharides.

12. The pharmaceutical composition according to claim 11, wherein said polysaccharide is heparin.

13. An agent for studying the growth regulation of blood and vascular cells or for studying HAPO receptor, wherein it comprises HAPO and/or mutant.

14. An antigen for preparing monoclonal antibodies or polyclonal antibodies against HAPO, wherein antigen is HAPO or the mutant thereof.

15. An antibody, wherein it is prepared using said antigen according to claim 14.

16. An agent for propagating vascular endothelial cells, and hematopoietic stem cells, wherein said agent comprises HAPO and/or the mutant thereof.

17. A medicament for propagating vascular endothelial cells or hematopoietic stem cells or for treating thrombocytopenia/panhematopenia, wherein said medicament is selected from HAPO, HAPO mutant and the pharmaceutical composition of according to any one of claim 8-11.
